# EUROPEAN PATENT APPLICATION

(11) **EP 0 770 349 A1**
(43) Date of publication of application: **02.05.1997**
(21) Application number: 96115041.4
(22) Date of filing: 19.09.1996
(51) Int. Cl.: A61B 5/00

(54) **Apparatus for monitoring patients**

(30) Priority: 19.09.1995 JP 240125/95; 30.10.1995 JP 281862/95
(71) Applicant: Akasaka, Noboru, Tokyo (JP); Akai, Koji, Kamakura-shi, Kanagawa-ken (JP)
(72) Inventor: Akasaka, Noboru, Tokyo (JP); Akai, Koji, Kamakura-shi, Kanagawa-ken (JP)
(74) Representative: Wagner, Karl H., Dipl.-Ing.

(57) **Abstract**

A monitoring apparatus (10,110) is wirelessly attached to a portion of a person's body for automatically obtaining the medical data of the person and transmitting the thus obtained data to a receiver. The apparatus (10,110) comprises means for non-invasively detecting, as the person's vital sign, said data including one or a combination of the pulse, pulse wave, electrocardiogram, temperature, blood pressure and SₚO₂ of the person via the interfacing surface between the apparatus and the contacted portion on the person's body, and means (18) for transmitting said detected data to said receiver (19) by means of wireless or optical communication medium such as infrared rays.

## Description

The present invention relates to an apparatus for remotely monitoring the physical condition of a patient who is at home or being taken care of in a hospital, and also for monitoring the health of elderly people. More particularly, the present invention relates to an apparatus attached to a part of a patient's body to enable remote monitoring by means of radio or optical transmission by for example radio or infrared rays.

As the ratio of elderly people in the population increases along with incumbent medical expenses, an apparatus for remotely monitoring the physical condition of a patient regardless of whether the patient is at home or is being taken care of at a hospital while keeping to a minimum the burden both on the patient's side and on the medical institute's side, is desired.

As prior art apparatuses, a bed-side type apparatus for use in a hospital or a transport type apparatus for use in an ambulance, which is in reality said bed-side type apparatus taken into and used in an emergency vehicle, are known. Though it may be possible to wirelessly transmit data by using these prior art monitoring apparatuses, electrodes of an electrocardiographic sensor, wires that connect said sensor to the electrodes, or various apparatuses as a tube for a sphygmomanometer must still be attached to a patient's body, thus restricting his/her activity and sometimes causing and discomfort. Accordingly, a patient monitoring apparatus is strongly desired that does not restrict overly a patient's activity and that enables the patient to live as normal a life as possible with his/her physical conditions being appropriately monitored.

A portable pendant-type apparatus is also known that is used to transmit an emergency signal that indicates the occurrence of a medical emergency. This type of apparatus, however, is designed on the premise that the patient can activate a switch by pulling it down. Thus if a medical emergency occurs suddenly and a patient is unable to operate a switch, the apparatus will not function at all. The present invention is aimed at remedying such a defect.

Japanese Patent Application No. 203,369/1995 discloses an invention called Medical System for Patients-at-Home. In accordance with the thus disclosed invention, a system which comprises a network formed from a group of home units each of which is set up at a plurality of patients' homes with at least one central unit being provided at a medical institute which functions as a local center for the patients-at-home in the community. The home unit as well as the central unit consists of a computer equipped with a high speed microprocessor (CPU) and an appropriate modem which allows bi-directional communication between each of the home units and the medical center, thereby exchanging information on a real time basis so that a physician or a nurse can provide the patient-at-home with instructions appropriate to his/her specific bodily condition, based on the medical information acquired via the system. This application is herein incorporated by reference.

In the above system, medical information is provided to the home unit from various measurement apparatus. The present invention is analogous to this system in that it relates to a measurement apparatus or monitoring apparatus that is attached to some portion of the patient's body without restricting his/her bodily activities and that it is capable of measuring/monitoring various bodily data in an appropriate and integrated manner. The present invention includes, among other things that are not included in the above incorporated system, an interface means by which medical data can be collected from the patient's body and which couples the acquired data to the home unit placed in the patient's home.

An object of the present invention is to provide a patient monitoring apparatus which minimizes restrictions to patient's movement and also discomfort caused by the apparatus and yet enables effective measurement of bodily data, thereby making it possible for an appropriate and integrated diagnosis to be made and, if necessary, for medical treatment to be initiated at an early stage.

In accordance with one feature of the present invention, a monitoring apparatus is provided which is wirelessly attached to a part of a person's body for automatically obtaining the medical data of the person and transmitting the thus obtained data to a receiver. The apparatus comprises means for non-invasively detecting, as the person's vital signs, said data including one or a combination of the pulse, pulse wave, electrocardiogram, temperature, blood pressure and SₚO₂ of the person via the interfacing surface between the apparatus and the contacted portion on the person's body, and means for transmitting said detected data to said receiver by means of wireless or optical communication medium such as infrared rays. The apparatus may further comprises means for comparing said detected data with a set of reference values indicative of the person's normal bodily condition and for displaying on a display of the apparatus or notifying said receiver of whether said detected data is within a normal range. In this apparatus, said non-invasive detecting means may comprise means for measuring SₚO₂ of the person by emitting infrared rays and/or visible light to a part of the body and detecting a return consisting of the reflected and diffused rays.

In accordance with another feature of the present invention, the above-mentioned apparatus may be appropriately formed so as to be inserted into one or both of external acoustic meatuses of the person's body. In this case, the apparatus is shaped as an earplug that has a hollow structure passing through the apparatus so that the person's hearing ability is maintained and said detecting means comprises one or more sensors or transducers mounted on the surface of the apparatus. The apparatus may further comprise means for enhancing the hearing ability by amplifying the sounds coming into the external acoustic meatus. The apparatus may be formed from a soft and flexible or shape memory material.

Fig. 1 is a block diagram illustrating the internal mechanism of the first embodiment of the patient monitoring apparatus in accordance with the present invention.

Fig. 2 is a perspective view of the first embodiment of the patient monitoring apparatus in accordance with the present invention.

Fig. 3 is a view of the reverse side that directly touches the patient's skin of the first embodiment of the patient monitoring apparatus in accordance with the present invention.

Fig. 4 is a cross sectional view of the second embodiment, worn into an external acoustic meatus, of the patient monitoring apparatus in accordance with the present invention.

Fig. 5 is a (partially transparent) perspective view of the second embodiment of the patient monitoring apparatus in accordance with the present invention.

Fig. 6 is a block diagram illustrating the internal mechanism of the second embodiment of the patient monitoring apparatus in accordance with the present invention.

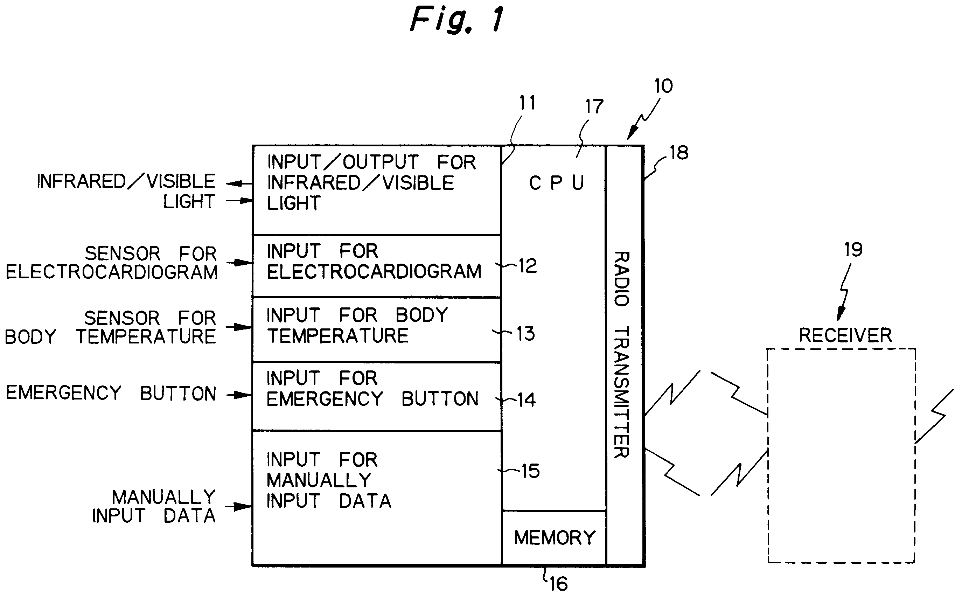

Preferred embodiments of the present invention will now be described by referring to the attached drawings. Referring to Fig. 1, a block diagram illustrating the internal mechanism of the first embodiment of the patient monitoring apparatus 10 in accordance with the present invention is provided. The patient monitoring apparatus 10 is in the form of a wrist watch and directly attached to a part of the patient's body, for example, on his/her wrist, ankle, finger, or arm by means of a band. The patient monitoring apparatus 10 comprises an infrared/visible light input/output for transmitting/receiving infrared light and visible light to measure the pulse, pulse waves and SₚO₂ of the patient; an input 12 from an electrocardiographic sensor; an input 13 from a body temperature sensor; input 14 from an emergency button; and an input 15 for manually inputting data such as results of urine examination and blood glucose values, etc. Each of these inputs 11-15 is connected to the central processing unit (CPU) 17, which is provided with infrared and visible light for measurement and a plurality of measured data from the above-mentioned input and then which supplies the data, after processing them or as they are, to the parental unit or receiving unit 19 via the radio transmitter 18.

The receiving unit 19, which is a "home unit" disclosed in the above incorporated Japanese application, is installed at the patient's house and has a computer incorporated therein so as to automatically function to transmit data via an appropriate modem. The data received by the unit 19 is transmitted through transmission medium to a center unit (not shown) installed at the medical institute which is in charge of the area. If the patient is being taken care of at a hospital and a patient monitoring apparatus 10 is attached to him/her, the receiving unit 19 is also installed at the hospital and transmits the processed or raw data to a center unit at the same hospital or at the central medical institute in charge of said hospital.

The CPU 17 is capable of, if necessary, having the input data and processed results temporarily stored in the memory 16. The CPU 17 calculates the blood pressure on the basis of the pulse and electrocardiographic data obtained via infrared detection and/or compares each of the input data with the reference value predetermined by a physician. If any of the input data is not within the predetermined range, the CPU 17 produces resultant information indicative of the result of the comparison. Whether the input data is to be transmitted as it is or after processed is selectively determined by means of the mode setting-up button 22. When desired, the processed results will be displayed on the display 20 by the CPU 17.

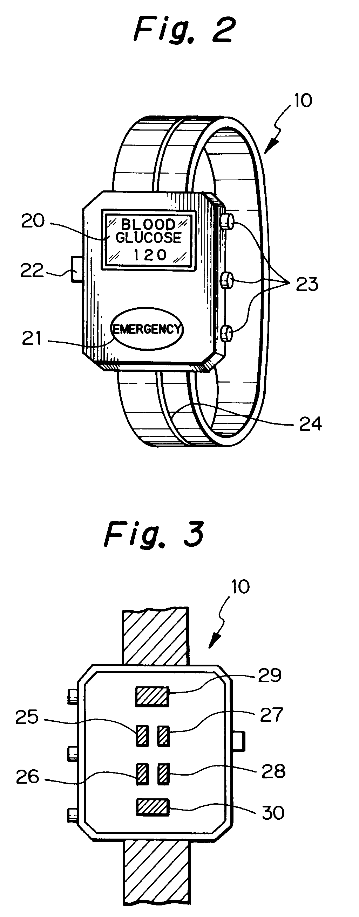

Referring to Fig. 2, a front perspective view of the first embodiment of the present patient monitoring apparatus 10 is shown, and is attached like a wrist watch. The button switch 22 is a mode set-up button used to switch between various modes. Each of a plurality of button switches 23 is used for the patient wearing this apparatus to manually input data such as blood glucose values, body weight, and results of urine examination. The emergency button switch 21, when activated by the patient in a medical emergency, have an emergency signal wirelessly or optically transmitted.

Next, referring to Fig. 3, the reverse side that directly touches the patient's skin in the first embodiment of the present patient monitoring apparatus 10 is shown. The infrared light produced by the infrared light transmitting unit 25 passes through a portion of the patient's body and after being reflected and/or diffused, it is measured by the infrared light detecting sensor 26. The light emitted from the visible light transmitting unit 27 also passes through a portion of the patient's body and is measured by the visible light detecting sensor 28. The CPU 17 calculates the oxygen saturation of arterial blood (SₚO₂), pulse and pulse wave from these data. The electrocardiographic sensor 29 detects the electrocardiogram of the patient. The detected electrocardiogram is amplified and its noise is removed at the electrocardiograph input unit 12 and CPU 17. Data indicative of the body temperature of the portion in touch with the body temperature sensor 30 is sent to the CPU 17 via the body temperature input unit 13.

Since all the sensors described above are mounted on the reverse side surface and the measurement of the various data is automatically performed, the patient is not physically restricted during detection of medical data and sending the detected data to the receiving unit 19 via an appropriate medium such as infrared light under a computer (CPU 17).

Notification of a medical emergency by the present apparatus comprises in two stages. In the first stage, the patient can notify medical staff at a medical institute with a center unit installed of his/her condition by activating the emergency button switch 21 on the apparatus 10. In response to the activation of this switch 21, an emergency signal is transmitted to the receiving unit by way of the wireless or optical communication function equipped with this apparatus 10. On receipt of this emergency signal from the patient, the medical institute will take emergency measures to cope with the situation.

The second stage, in accordance with this apparatus 10 already disclosed in the above mentioned Japanese patent application, and involves continuous detection of the patient's vital signs. However, in this case, vital signs are automatically and continuously detected by using the wrist watch type apparatus 10 rather than the more cumbersome means provided by the foregoing application. In order to more fully realize the vital sign detection function than in the case of the above first stage, the apparatus 10 continuously detects the patient's vital signs on a twenty-four hour basis. Preferably, vital signs to be detected include the pulse detected in the patient's wrist. As far as any predetermined vital sign can be detected in an ordinary manner, electrical signals continue to be transmitted from the monitoring apparatus 10. If the patient stays within a predetermined distance from the home (receiving) unit 19, the home unit 19 is capable of receiving signals. The CPU incorporated in the home unit will not take any action as long as the electrical signals continue since the continuous detection of signals assumes that the patient is in a normal condition. However, once signals cease to be detected, the home unit will immediately transmit an emergency signal to the medical institute and notify the medical staff there of the occurrence of an emergency without any investigation of the patient's physical condition. Although signal failure may be due to an electrical fault in the apparatus 10 or any other extraordinary event as well as the occurrence of a physical disorder, the present apparatus 10 will without exception, decide on a general fail-safe principle that a medical emergency has occurred, thus enabling a critical response to be taken.

The pendant type apparatus belonging to the prior art can be effective only if the patient actuates the switch him/herself by pulling the strong attached thereto, for example. Whereas the present apparatus will always be effective even if the occurrence of medical emergency is so sudden and unexpected that the patient him/herself does not have the chance or strength to pull the switch. Thus the present apparatus 10 is more reliable than the prior art apparatuses which require the activation of a switch by the patient.

In order to facilitate this function, the home unit can be set to an absent mode to avoid an emergency signal being wrongly transmitted to the center unit, or the emergency notification function can be turned off.

Depending on the patient's condition, ETCO₂ can be detected as a vital sign, wherein the internal mechanism will be the same except for a difference in the detector thereof.

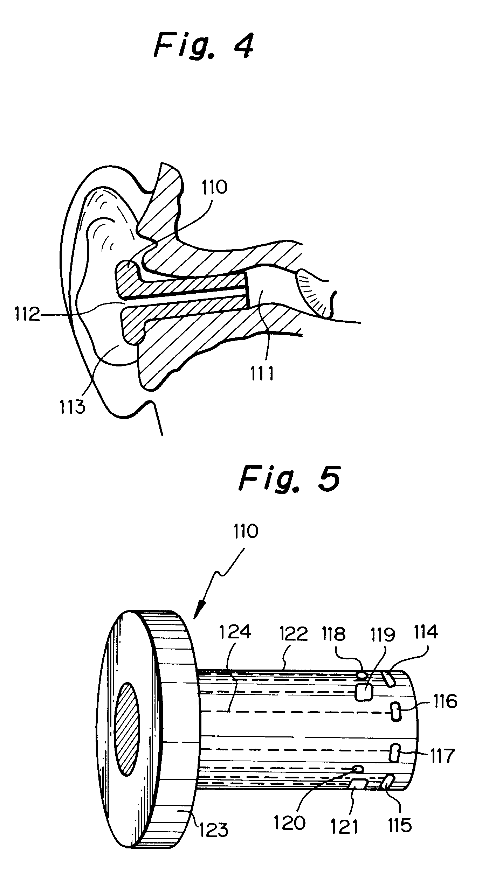

Referring to Fig. 4, which illustrates a cross sectional view of the second embodiment of the patient monitoring apparatus in accordance with the present invention. another type of the present apparatus will be described. As shown in the figure, the apparatus 110 is shaped like an earplug and worn in an external acoustic meatus of the patient. Preferably, in order to maintain the hearing capacity, the apparatus 110 has a hollow portion 112 passing through at the center of this cylindrical-shaped apparatus 110. The outside surface of the apparatus 110 consists of a flexible and soft material to prevent discomfort while the sensors and transducers mounted on the surface comfortably contact the skin in the external acoustic meatus of the patient.

Fig. 5 is a (partially transparent) perspective view of this earplug type second embodiment of the present patient monitoring apparatus. On the surface of the apparatus 110 in touch with the skin in the external acoustic meatus, electrodes 114, 115 for the detection of the electrocardiograph, a transducer 116 for the detection of pulse wave (pressure) and a sensor 117 for the body temperature are mounted.

In addition, the infrared light emitted by the infrared light transmitting unit 118 passes through a portion of the patient's body and after being reflected and/or diffused, returned light is measured by the infrared light detecting sensor 119. Similarly the light emitted from the visible light transmitting unit 120 also passes through a portion of the patient's body and is measured by the visible light detecting sensor 121. The sensors 114, 115, 117, 119, 121 and transducer 116 are located on the surface of such a portion 122 of the apparatus 110 inserted into the ear. The surface of this inserted portion on which the plurality of sensors are mounted is preferably smooth so that the patient wearing the apparatus is not subject to any discomfort. The sensors and transducer are connected to the control and transmitting unit 123 via an lead 124 on which the detected data will be transferred.

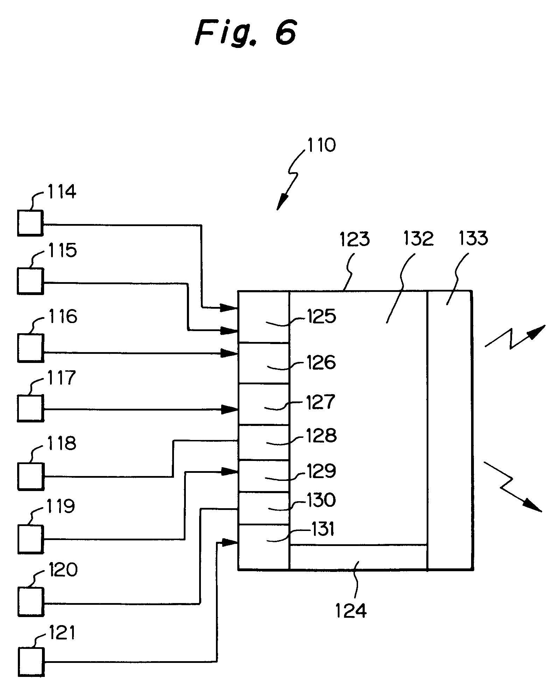

Fig. 6 is a block diagram illustrating the internal mechanism of this second embodiment. As shown, the electrocardiographic signal detected with the electrocardiogram detection electrodes 114, 115 is provided to the processor 132 via the electrocardiogram input unit 125. The pulse wave signal (by way of pressure) detected with the pressure transducer 116 is provided to the processor 132 via pressure input unit 126. The temperature signal detected with the temperature sensor 117 is provided to the processor via the temperature input unit 127. The infrared light is emitted from the infrared light output unit 128 and then the emission unit so as for the oxygen saturation of arterial blood (SₚO₂), pulse wave and blood pressure to be measured. The emitted infrared light returns from the patient's body and is measured by the infrared light detecting sensor 119. Similarly the visible light emitted from the visible light transmitting unit 120 also passes through a portion of the patient's body and is measured by the visible light detecting sensor 121. On the basis of the received amount of the infrared and/or visible light, SₚO₂, pulse wave and blood pressure are calculate.

In detecting and measuring the electrocardiogram, in particular, it may be preferable to attach an appropriate electrode in each of the patient's external acoustic meatuses and to connect them each other so as to correlate the data from both of them.

The processor 132 is powered by an exchangeable Lithium battery and has a microprocessor (not shown) incorporated therein and the microprocessor performs the above described calculation using the data detected. Incidentally by performing the measurement by this apparatus 110 continuously and/or once in every predetermined time period, it is possible to collect a series of medical data and construct a medical database about a specific group of patients over some time period. The obtained data will be wirelessly sent to the home unit.

Although the apparatus 110 has a hollow portion at the center of its cylindrical portion, it may be possible that the hearing capacity of the person wearing it will be worse than otherwise. In such a case, conventional technique employed in an earplug type hearing aid will also be used in the apparatus 110. For example, a small microphone is mounted to transmit sound wave signals and to amplify them.

The emergency notification function of this earplug type second embodiment of the present invention is constructed similarly to the case with the first embodiment.

While several embodiments of the present invention have been shown and described, alternate embodiments will be apparent to those skilled in the art and are within the intended scope of the present invention. Therefore, the invention is to be limited only by the following claims.

## Claims

1. A monitoring apparatus wirelessly attached to a portion of a person's body for automatically obtaining the medical data of the person and transmitting the thus obtained data to a receiver, comprising:
means for non-invasively detecting, as the person's vital sign, said data including one or a combination of the pulse, pulse wave, electrocardiogram, temperature, blood pressure and/or SₚO₂ of the person via the interfacing surface between the apparatus and the contacted portion on the person's body, and
means for transmitting said detected data to said receiver by means of wireless or optical communication medium such as infrared rays.

2. The apparatus recited in claim 1 further comprising means for comparing said detected data with a set of reference values indicative of the person's normal bodily condition and for displaying on a display of the apparatus or notifying said receiver of whether said detected data is within a normal range of the values.

3. The apparatus recited in claim 1 wherein said non-invasive detecting means comprises means for measuring SₚO₂ of the person by emitting infrared rays and/or visible light to a portion on the body and detecting a return consisting of the reflected and diffused rays and light.

4. The apparatus recited in claim 1 wherein said vital sign continues to be detected and further comprising means for automatically transmitting an emergency signal to said receiver upon detecting the abnormality of the vital sign or upon an intentional activation of a switch by the person.

5. The apparatus recited in claim 1 further comprising means for manually inputting additional medical data such as the result of a urine examination, weight and blood glucose value.

6. The apparatus recited in any one of claims 1 to 5 wherein said apparatus is appropriately formed so as to be attached to a wrist, ankle, finger or arm of the person's body.

7. The apparatus recited in any one of claims 1 to 5 wherein said apparatus is appropriately formed so as to be inserted into one or both of external acoustic meatuses of the person's body.

8. The apparatus recited in claim 7 wherein the apparatus is shaped as an earplug that has a hollow structure passing through the apparatus so that the person's hearing ability is maintained and said detecting means comprises one or more sensors or transducers mounted on the surface of the apparatus.

9. The apparatus recited in claim 8 further comprising means for enhancing the hearing ability by amplifying the sounds coming into the external acoustic meatus.

10. The apparatus recited in any one of claims 7 to 9 wherein the apparatus is formed from a soft and flexible or shape memory material.
